# FASCICULE DE BREVET EUROPEEN

(11) **EP 0 680 952 B1**
(45) Date de publication et mention de la délivrance du brevet: **28.07.1999**
(21) Numéro de dépôt: 95401011.2
(22) Date de dépôt: 02.05.1995
(51) Int. Cl.: C07D 211/90

(54) **Procédé d'obtention de 1,4-dihydropyridines optiquement pures**
Verfahren zur Herstellung von optisch reinen 1,4-Dihydropyridinen
Process for the preparation of optically pure 1,4-dihydropyridines

(30) Priorité: 05.05.1994 FR 9405548
(43) Date de publication de la demande: 08.11.1995
(73) Titulaire: LABORATOIRE L. LAFON, 94701 Maisons Alfort (FR)
(72) Inventeur: Laurent, Philippe, F-69600 Oulins (FR)
(74) Mandataire: Le Guen, Gérard

(56) Documents cités:
- EP-A- 0 273 349
- EP-A- 0 383 320
- EP-A- 0 591 773

## Description

La présente invention concerne un procédé d'obtention de 1,4-dihydropyridines optiquement pures.

On sait que les 1,4-dihydropyridines de formule : 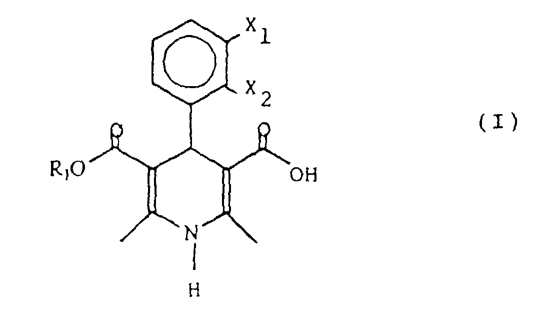 dans laquelle R₁ est un groupe alkyle en C₁-C₄,
et l'un des groupes X₁ et X₂ représente un groupe nitro, fluoro, chloro ou trifluorométhyle et l'autre un atome d'hydrogène ou à la fois X₁ et X₂ représentent un groupe chloro,
constituent une classe de composés ayant un grand intérêt pour la synthèse de dérivés utiles en thérapeutique. Comme exemples de tels dérivés on peut citer la nicardipine et la nifedipine.

Or tous ces composés présentent un centre d'asymétrie et sont sous forme d'isomères. Il est déterminant de pouvoir séparer les isomères ou de les préparer sous une forme pratiquement optiquement pure.

Or il n'existe pas à l'heure actuelle de méthode de préparation simple et économique des énantiomères des dihydropyridines de formule I.

Les méthodes actuellement connues sont basées sur la séparation de diastéréoisomères par différentes voies :
- utilisation de la chromatographie préparative sur colonne chirale, technique ne pouvant pas être encore industrialisée, et faisant appel à des phases stationnaires extrêmement coûteuses (M. Kajino et al., Chem. Pharm. Bull. 37(8) 2225-2228 (1989).
- blocage de l'azote de la dihydropyridine par des groupements éthoxyméthyle ou benzyle, et salification de la fonction acide de la dihydropyridine par des bases chirales du type cinchonine ou cinchonidine. Les sels des deux diastéréoisomères ainsi obtenus sont séparés par cristallisation fractionnée. Le retour à l'acide dédoublé (R ou S) se fait par acidification du sel, suivi de l'hydrolyse du groupe éthoxyméthyle, ou de l'hydrogénation catalytique du groupe benzyle. Ce procédé fait appel à des bases organiques extrêmement coûteuses et particulièrement toxiques. Il faut donc être certain de les avoir totalement éliminées pour la suite des opérations. Par ailleurs, le procédé est long et utilise des méthodes (par exemple l'hydrogénation), qui s'ajoutant au reste ne peuvent qu'aggraver le prix de revient de telles molécules (T. Shibanuma et al., Chem. Pharm. Bull. 28, 2809 (1980) - K. Muto et al., Arzneim. Forsch/Drug Res. 38 (II), 11a (1988).
- hydrolyse enzymatique sélective d'ester carbamoylméthyle de la dihydropyridine, d'accès difficile et coûteux. De plus, les enzymes utilisables sont des protéases et notamment une SEAPROSE-S, de coût exorbitant (Y. Hirose et al., Tetrahedron Letters, 34, 37, 5915-5918, 1993).

La présente invention est basée sur la constatation que les sels de calcium de l'acide lactique chiral (R ou S) estérifié par une dihydropyridine racémique formaient deux diastéréoisomères dont les vitesses de cristallisation dans l'eau sont extrêmement différentes. Cette propriété remarquable a été mise à profit pour mettre au point une méthode de résolution des énantiomères de dihydropyridine ayant un centre d'asymétrie.

La présente invention a ainsi pour objet un procédé d'obtention d'une 1,4-dihydropyridine de formule Ia ou Ib : 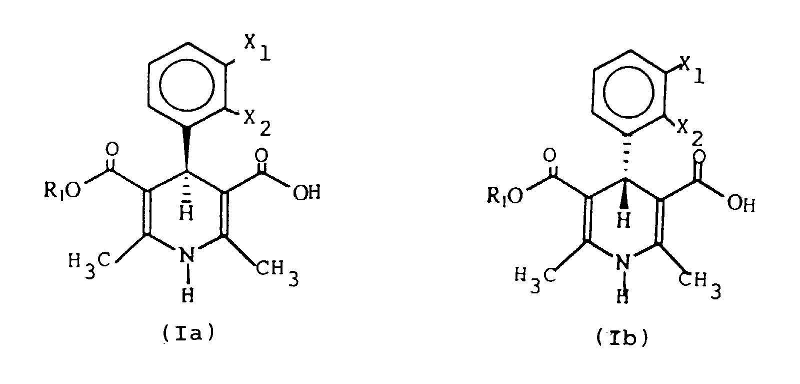 dans laquelle R₁ est un groupe alkyle en C₁-C₄,
et l'un des groupes X₁ et X₂ représente un groupe nitro, fluoro, chloro ou trifluorométhyle et l'autre un atome d'hydrogène ou à la fois X₁ et X₂ représentent un groupe chloro,
qui comprend la cristallisation d'un sel de calcium d'un acide lactique estérifié par une 1,4-dihydropyridine racémique,de formule 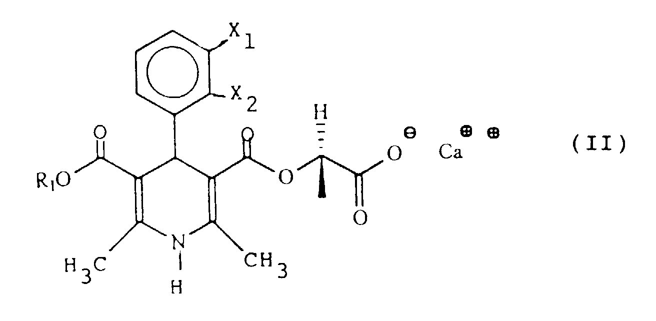 de façon à séparer des cristaux de sel de calcium de formule : 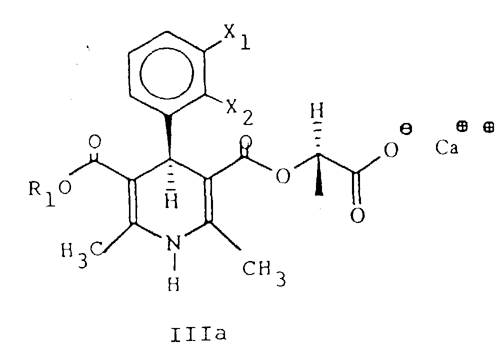 ou 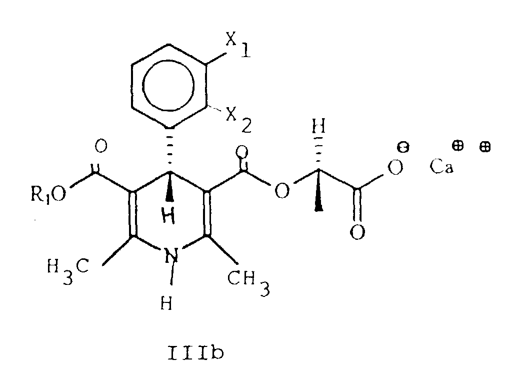
et l'hydrolyse du sel cristallisé IIIa ou IIIb pour obtenir respectivement une 1,4-dihydropyridine de formule Ia ou Ib.

La préparation des sels de calcium de formule Il peut être réalisée par :
a) la réaction d'un acétoacétate de (R) ou (S) lactate d'alkyle de formule 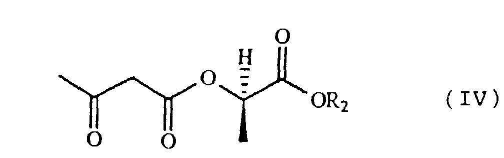 dans laquelle R₂ est un groupe alkyle en C₁ à C₆,
   d'un aminocrotonate d'alkyle de formule 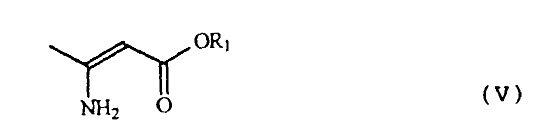 dans laquelle R₁ a la signification donnée à la revendication 1,
   et d'un aldéhyde aromatique de formule : 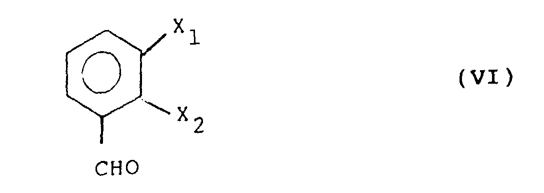 dans laquelle X₁ et X₂ ont la signification donnée ci-dessus,
   pour obtenir un ester d'acide lactique de formule : 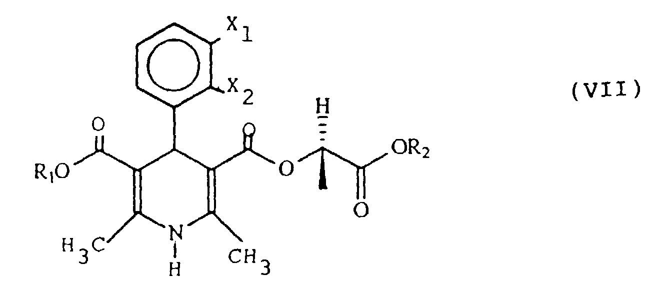
b) la saponification du composé de formule VII dans des conditions douces pour obtenir un composé de formule : 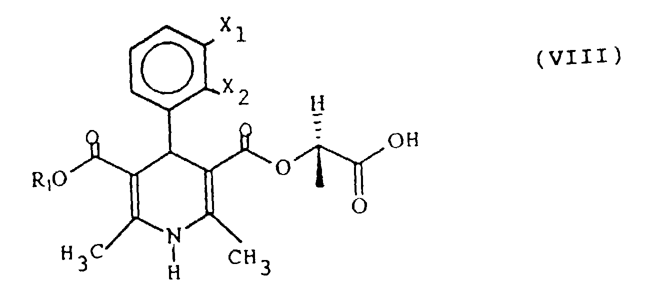
c) la transformation du composé de formule VIII en sel de calcium de formule II.

Ainsi, la dihydropyridine peut être synthétisée à partir d'un acétoacétate d'ester lactique R ou S, d'aminocrotonate d'alkyle et de 3-nitro-benzaldéhyde. L'ester lactique est saponifié de façon douce puis, par exemple, par l'intermédiaire d'hydrogénocarbonate de sodium et de chlorure de calcium, il est transformé en sel de calcium. L'un des deux diastéréoisomères cristallise au bout de quelques minutes. Il est séparé par filtration, lavé puis hydrolysé afin de retourner à l'acide. L'autre diastéréoisomère cristallise après plusieurs jours, mais il est très impur.

Il suffit de choisir l'ester lactique adéquat pour obtenir le composé de formule Ia ou Ib désiré. Ainsi, le (R)-(+)-lactate d'alkyle (par exemple le (R)-(+)-lactate d'isobutyle) conduit à une (R)-(-)-dihydropyridine de formule Ia, et le S-(-)-lactate d'alkyle (par exemple le (S)-(-)-lactate d'éthyle) conduit à l'acide (S)-(+)-dihydropyridine de formule Ib.

Les exemples suivants illustrent l'invention.

### EXEMPLE 1

### Préparation du monométhylester de l'acide (S)-(+)-2,6-diméthyl-4-(3-nitro-phényl)-1,4-dihydropyridine-3,5-dicarboxylique

A 202 grammes (1 mole) d'acétoacétate de (S)-(-)-lactate d'éthyle on ajoute 115 grammes (1 mole) d'aminocrotonate de méthyle et 151 grammes (1 mole) de 3-nitrobenzaldéhyde. On chauffe entre 110 et 120° C pendant 4 heures. On refroidit et dilue dans 1 litre de méthanol. On alcalinise sans dépasser 20° C par une mole de lessive de soude (d=1,33). On agite 10 minutes en maintenant la température au-dessous de 20° C.

On dilue à l'eau, acidifie et extrait au dichlorométhane. On lave la solution organique par un litre d'une solution aqueuse molaire d'hydrogénocarbonate de sodium. Sous vive agitation, on déplace le sel de sodium formé précédemment par 111 grammes (1 mole) de chlorure de calcium dissous dans 100 ml d'eau.

Le sel de calcium précipite. On filtre et écarte les eaux mères qui renferment préférentiellement le diastéréoisomère opposé.

Les cristaux sont lavés à l'eau bouillante puis séchés. On obtient 120 grammes de sel de calcium (0,27 mole, 54 % du diastéréoisomère attendu).

Après saponification en milieu anhydre, on isole 85 grammes de produit brut, qui purifié conduit à 60 grammes de produit (0,18 mole, 36 % de l'énantiomère attendu).
[α]_{D} = + 19,4 (c 1,0, acétone), (% ee) = 98,0

### EXEMPLE 2

### Préparation du monométhylester de l'acide (R)-(-)-2,6-diméthyl-4-(3-nitro-phényl)-1,4-dihydropyridine-3,5-dicarboxylique

A 230 grammes (1 mole) d'acétoacétate de (R)-(+)-lactate d'isobutyle on ajoute 115 grammes (1 mole) d'aminocrotonate de méthyle et 151 grammes (1 mole) de 3-nitrobenzaldéhyde. On chauffe entre 110° C et 120° C pendant 4 heures. On refroidit et dilue dans 1 litre de méthanol. On alcalinise sans dépasser 20° C par une mole de lessive de soude (d=1,33). On agite 10 minutes en maintenant la température au-dessous de 20° C.

On dilue à l'eau, acidifie et extrait au dichlorométhane. On lave la solution organique par un litre d'une solution aqueuse molaire d'hydrogénocarbonate de sodium. Sous vive agitation on déplace le sel de sodium formé précédemment par 111 grammes (1 mole) de chlorure de calcium dissous dans 100 ml d'eau.

Le sel de calcium précipite. On filtre et écarte les eaux mères qui renferment essentiellement le diastéréoisomère opposé.

Les cristaux sont lavés à l'eau bouillante puis séchés. On obtient 140 grammes de sel de calcium (0,315 mole, 63 % du diastéréoisomère attendu).

Après saponification en milieu anhydre, on isole 102 grammes de produit brut, qui purifié conduit à 82 grammes (0,247 mole, 49,4 % de l'énantiomère attendu).
[α]_{D} = -20 (c 1,0, acétone), (% ee) = 96,8.

## Revendications

1. Procédé d'obtention d'une 1,4-dihydropyridine de formule Ia ou Ib 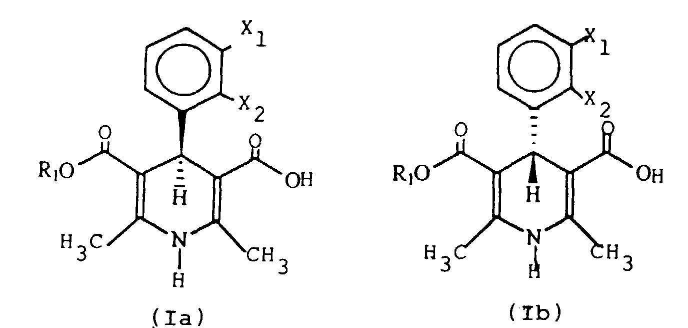 dans laquelle R₁ est un groupe alkyle en C₁-C₄,
et l'un des groupes X₁ et X₂ représente un groupe nitro, fluoro, chloro ou trifluorométhyle et l'autre un atome d'hydrogène ou à la fois X₁ et X₂ représentent un groupe chloro,
qui comprend la cristallisation d'un sel de calcium d'un acide lactique estérifié par une 1,4-dihydropyridine racémique,de formule : 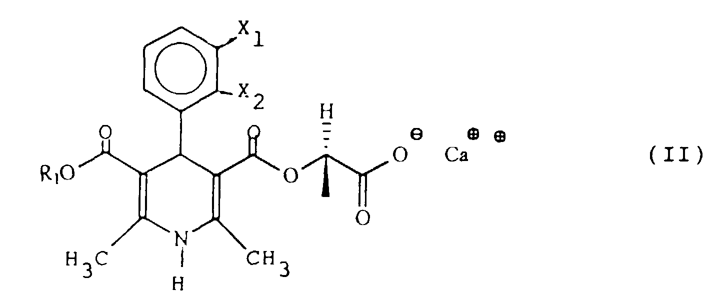 de façon à séparer des cristaux de sel de calcium de formule : 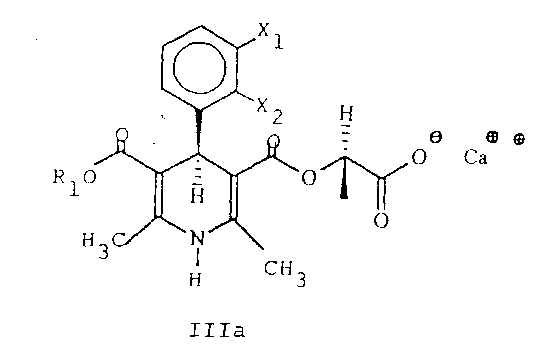 ou 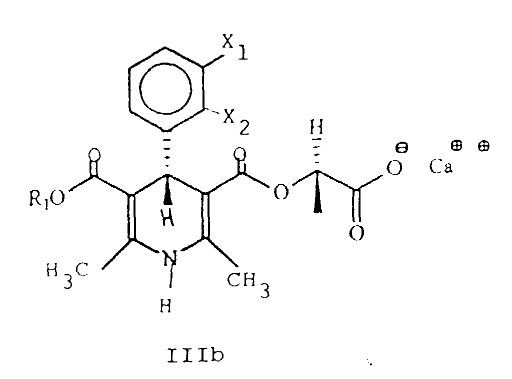
et l'hydrolyse du sel cristallisé IIIa ou IIIb pour obtenir respectivement une 1,4-dihydropyridine de formule Ia ou Ib.

2. Procédé selon la revendication 1, qui comprend, pour la préparation du sel de calcium de formule II :
a) la réaction d'un acétoacétate de (R) ou (S) lactate d'alkyle de formule 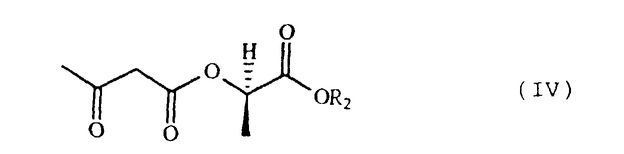 dans laquelle R₂ est un groupe alkyle en C₁ à C₆, d'un aminocrotonate d'alkyle de formule 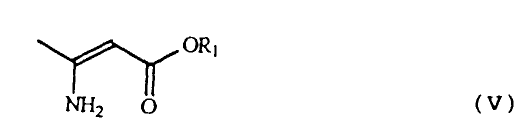 dans laquelle R₁ a la signification donnée à la revendication 1,
et d'un aldéhyde aromatique de formule : 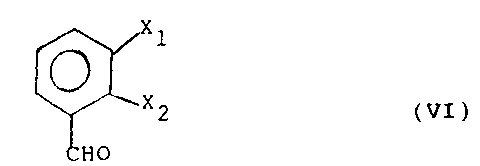 dans laquelle X₁ et X₂ ont la signification donnée à la revendication 1,
pour obtenir un ester d'acide lactique de formule : 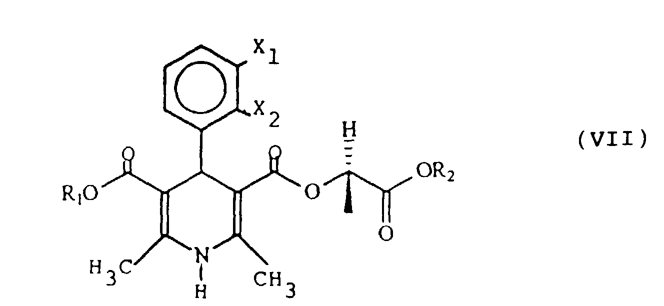
b) la saponification du composé de formule VII dans des conditions douces pour obtenir un composé de formule : 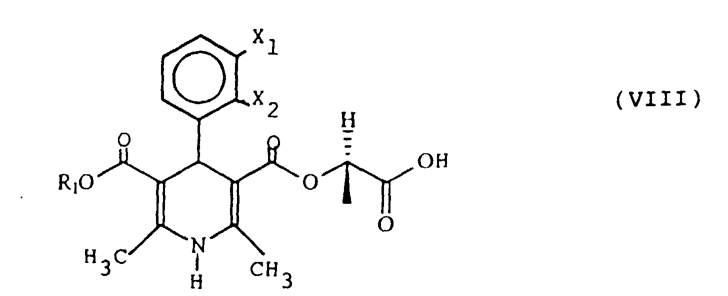
c) la transformation du composé de formule VIII en sel de calcium de formule II.

3. Procédé selon la revendication 2, dans lequel on utilise comme composé de formule IV un acétoacétate de (R)-(+)-lactate d'alkyle et l'on obtient une (R)-(-)-1,4-dihydropyridine de formule Ia.

4. Procédé selon la revendication 2, dans lequel on utilise comme composé de formule IV un acétoacétate de (S)-(-)-lactate d'alkyle et l'on obtient une (S)-(+)-1,4-dihydropyridine de formule Ib.

## Patentansprüche

1. Verfahren zur Herstellung eines 1,4-Dihydropyridins der Formel Ia oder Ib, 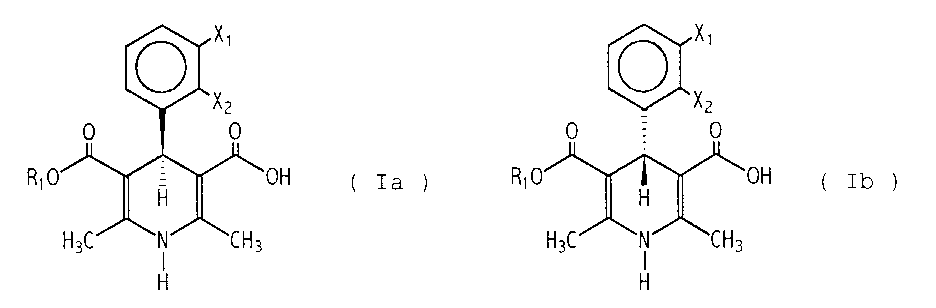
worin R₁ eine C₁-C₄-Alkylgruppe, und eine der Gruppen X₁ und X₂ eine Nitro-, Fluor-, Chlor- oder Trifluormethylgruppe und die andere ein Wasserstoffatom ist, oder X₁ und X₂ zugleich eine Chlorgruppe repräsentieren,
das aus der Kristallisation eines Calciumsalzes einer mit einem razemischen 1,4-Dihydropyridin veresterten Milchsäure der Formel 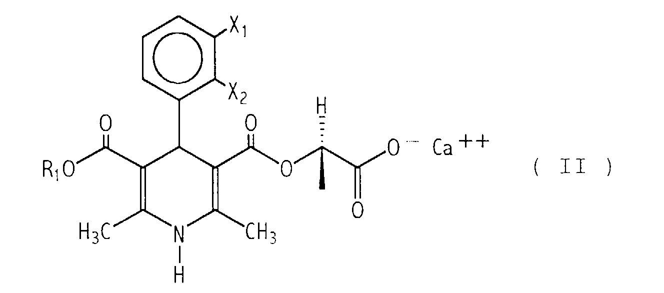 besteht, um Kristalle des Calciumsalzes mit folgender Formel voneinander zu trennen: 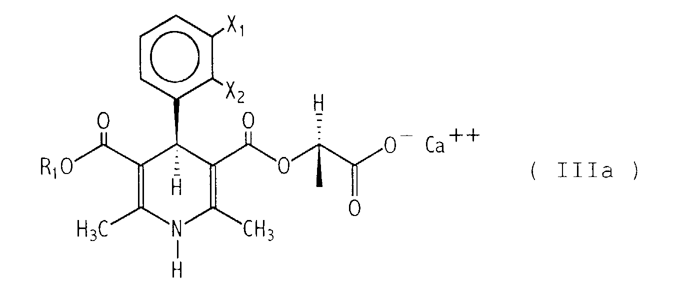 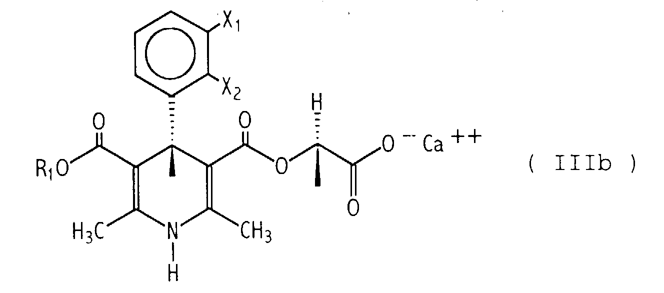
und der Hydrolyse des kristallisierten Salzes IIIa oder IIIb, beziehungsweise um ein 1,4-Dihydropyridin der Formel Ia oder Ib zu erhalten.

2. Verfahren nach Anspruch 1, das zur Herstellung des Calciumsalzes der Formel II besteht in:
a) der Reaktion eines Acetoacetats des (R)- oder (S)-Alkyllactats der Formel: 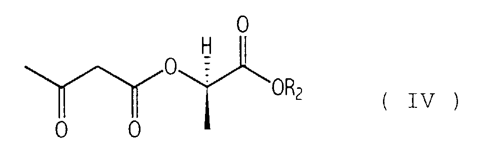 in dem R₂ eine C₁-C₆-Alkylgruppe eines Alkylaminocrotonats ist der Formel: 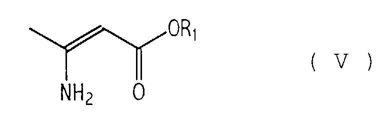 in der R₁ die Bedeutung gemäß Anspruch 1 hat,
mit einem aromatischen Aldehyd der Formel: 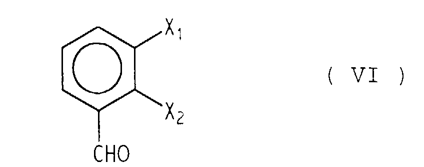 in dem X₁ und X₂ die Bedeutung gemäß Anspruch 1 haben,
um einen Milchsäureester zu erhalten mit der Formel: 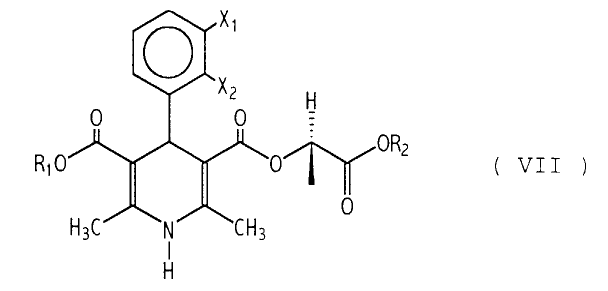
b) der Verseifung der Verbindung mit der Formel VII unter milden Bedingungen, um eine Verbindung zu erhalten mit der Formel: 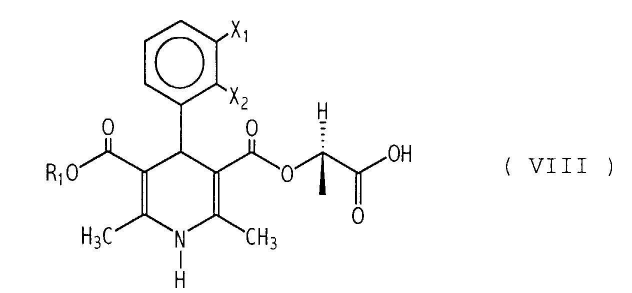
c) der Umwandlung der Verbindung mit der Formel VIII in ein Calciumsalz mit der Formel II.

3. Verfahren nach Anspruch 2, bei dem man als Verbindung mit der Formel IV ein Acetoacetat des (R)-(+)-Alkyllactats verwendet und ein (R)-(-)-1,4-Dihydropyridin mit der Formel Ia erhält.

4. Verfahren nach Anspruch 2, bei dem man als Verbindung mit der Formel IV ein Acetoacetat des (S)-(-)-Alkyllactats verwendet und ein (S)-(+)-1,4-Dihydropyridin mit der Formel Ib erhält.

## Claims

1. Process for producing a 1,4-dihydropyridine of formula Ia or Ib 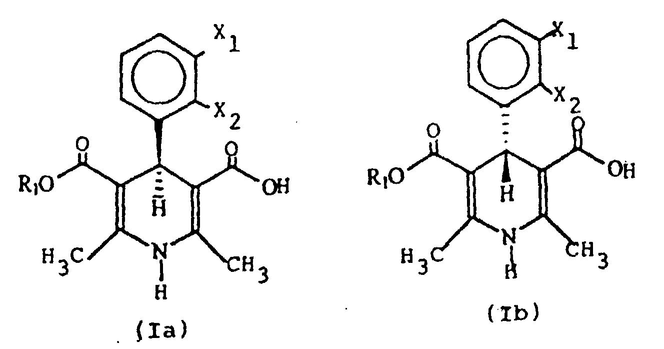
in which R¹ is a C₁-C₄ alkyl group,
and one of the groups X₁ and X₂ represents a nitro, fluoro, chloro or trifluoromethyl group and the other represents a hydrogen atom, or X₁ and X₂ both represent a chloro group,
which comprises the crystallization of a calcium salt of a lactic acid esterified with a racemic 1,4-dihydropyridine of formula 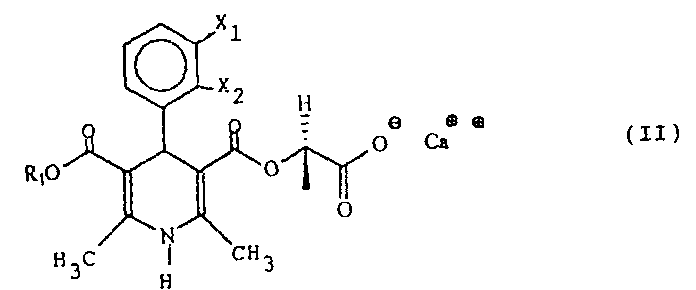 so as to separate out calcium salt crystals of formula: 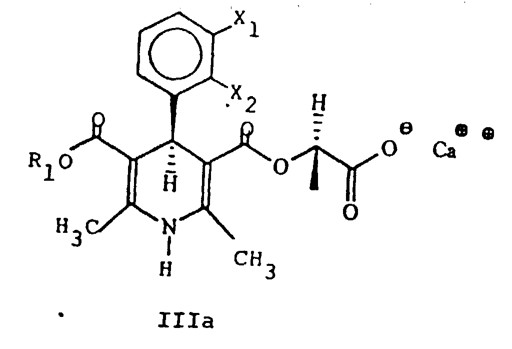 or 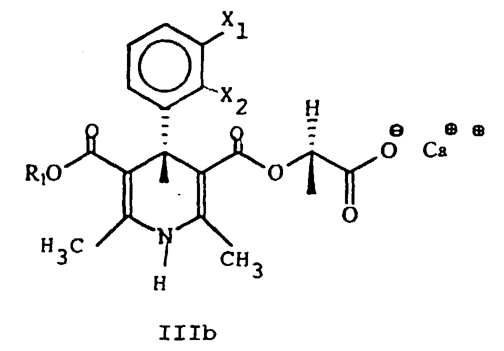
and hydrolysis of the crystallized salt IIIa or IIIb in order to obtain a 1,4-dihydropyridine of formula Ia or Ib respectively.

2. Process according to Claim 1, which comprises, for the preparation of the calcium salt of formula II:
a) the reaction of an acetoacetate of alkyl (R) or (S) lactate of formula 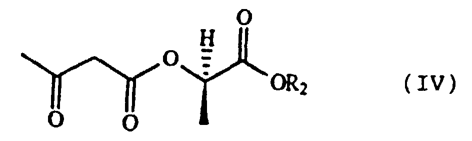 in which R₂ is a C₁ to C₆ alkyl group,
of an alkyl aminocrotonate of formula 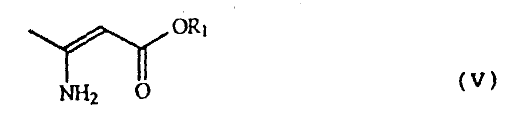 in which R₁ has the meaning given in Claim 1,
and of an aromatic aldehyde of formula: 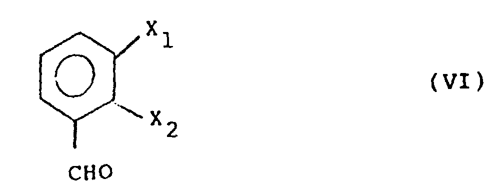 in which X₁ and X₂ have the meaning given above,
in order to obtain a lactic acid ester of formula: 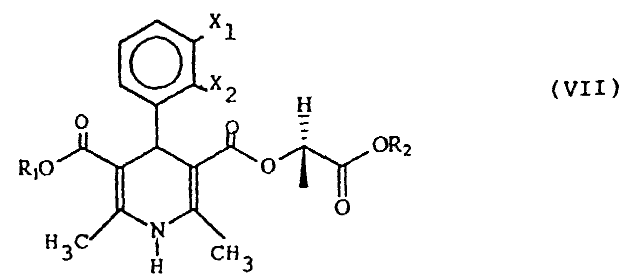
b) saponification of the compound of formula VII under mild conditions, to obtain a compound of formula: 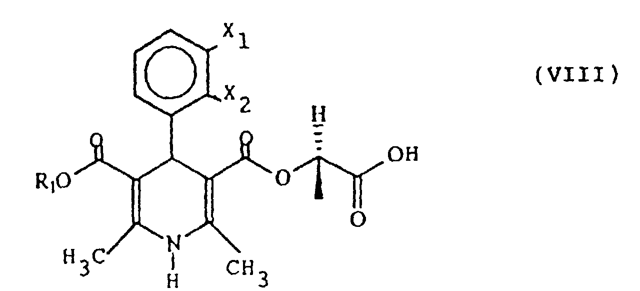
c) conversion of the compound of formula VIII into the calcium salt of formula II.

3. Process according to Claim 2, in which an acetoacetate of alkyl (R)-(+)-lactate is used as compound of formula IV, and an (R)-(-)-1,4-dihydropyridine of formula Ia is obtained.

4. Process according to Claim 2, in which an acetoacetate of alkyl (S)-(-)-lactate is used as compound of formula IV, and an (S)-(+)-1,4-dihydropyridine of formula Ib is obtained.
